# EUROPEAN PATENT APPLICATION

(11) **EP 2 491 907 A1**
(43) Date of publication of application: **29.08.2012**
(21) Application number: 10824812.1
(22) Date of filing: 08.10.2010
(51) Int. Cl.: A61F 13/15, A61F 13/49

(54) **METHOD AND DEVICE FOR REDUCING THICKNESS OF ABSORPTION BODY**

(30) Priority: 19.10.2009 JP 2009240708
(71) Applicant: Uni-Charm Corporation, Ehime 799-0111 (JP)
(72) Inventor: OGASAWARA, Yoshikazu, Kanonji-shi Kagawa 769-1602 (JP); YANO, Takanori, Kanonji-shi Kagawa 769-1602 (JP); ISHIKAWA, Masahiko, Kanonji-shi Kagawa 769-1602 (JP)
(74) Representative: Eke, Philippa Dianne
(86) International application number: PCT/JP2010/067735
(87) International publication number: WO 2011/048963

(57) **Abstract**

A method which makes a thickness of an absorbent body, including a liquid absorbent fiber and a superabsorbent polymer, thin, the method includes (1) transporting the absorbent body by moving a holding surface while holding the absorbent body on the holding surface with suction air from a suction hole on the holding surface, and (2) making the thickness of the absorbent body thin by sucking a belt member towards the holding surface using the suction air from the suction hole, in a predetermined range of a travel path of the holding surface, the belt member being arranged opposing the holding surface along the travel path and being arranged movably along the travel path over the predetermined range of the travel path, and sandwiching the absorbent body between the holding surface and the belt member.

## Description

### Technical Field

The present invention relates to a method and an apparatus that makes thin the thickness of an absorbent body for an absorbent article such as a disposable diaper.

### Background Art

Conventionally, a disposable diaper and a sanitary napkin are known as absorbent articles that absorb liquid such as bodily waste fluid. Such an absorbent article includes as a component part an absorbent body that absorbs liquid, and this absorbent body is formed by molding into a predetermined shape a liquid absorbent fiber such as pulp fiber made by mixing therein superabsorbent polymer (a high molecular weight polymer having a high liquid holding ability and that swells and the like by absorbing liquid).

On the other hand, with a view to providing a better appearance when clothes are worn and decreasing the sense of bulkiness, there is a need to make thinner an absorbent article such as a diaper and a sanitary napkin.

In regards to this point, PTL 1 discloses, in a method that makes an absorbent body thin, passing through and sandwiching and pressing an absorbent body in between a pair of rollers which are opposed to each other and rotate.

### Citation List

### Patent Literature

PTL 1: Japanese Translation of PCT International Application No. 2005-513288

### Summary of Invention

### Technical Problem

The method in PTL 1, however, is for sandwiching and pressing the absorbent body with a pair of rollers; therefore when sandwiching and pressing the absorbent body, the rollers are in line-contact with the absorbent body and will squash the absorbent body. Then, at that time, because of the high compression force of the line-contact, granular superabsorbent polymer in the absorbent body will also be excessively squashed and harden. As a result, there is a possibility that the absorbent body as a whole will become firm, and will give the wearer a sense of discomfort when wearing the absorbent article.

Further, in the case where a distribution density of the superabsorbent polymer in the absorbent body is varied, in a region with high distribution density, a large number of superabsorbent polymers also get excessively crushed and harden, and therefore the hardness of this region becomes higher than that of other regions in the absorbent body. In other words, inconsistency of hardness is made apparent or is enlarged, and thus this also becomes the reason of the sense of discomfort when wearing as described above.

Further, in an area where a large number of superabsorbent polymers are excessively squashed, such polymers connect flatly and spread largely in a surface that intersects the thickness direction, and gel blocking easily happens (a phenomenon where the superabsorbent polymer that has absorbed liquid swells and connects as a wall, and liquid is inhibited from permeating in the thickness direction of the absorbent body), in other words there is also a possibility that inhibition of liquid absorption occurs.

### Solution to Problem

An advantage of some aspects of the present invention is to provide a method which makes a thickness of an absorbent body, which can be made thin in thickness, thin, by suppressing excessive squashing of superabsorbent polymer, and an apparatus therefor.

An aspect of the invention is a method which makes a thickness of an absorbent body, including a liquid absorbent fiber and a superabsorbent polymer, thin, the method comprising:
transporting the absorbent body by moving a holding surface while holding the absorbent body on the holding surface with suction air from a suction hole on the holding surface; and
making the thickness of the absorbent body thin by
   sucking a belt member towards the holding surface using the suction air from the suction hole, in a predetermined range of a travel path of the holding surface, the belt member being arranged opposing the holding surface along the travel path and being arranged movably along the travel path over the predetermined range of the travel path, and
   sandwiching the absorbent body between the holding surface and the belt member.

Further, an apparatus which makes a thickness of an absorbent body, including a liquid absorbent fiber and a superabsorbent polymer, thin, the apparatus comprising:
a first apparatus which transports the absorbent body by having a holding surface which moves while holding the absorbent body on the holding surface with suction air from a suction hole on the holding surface; and
a second apparatus which makes the thickness of the absorbent body thin by having a belt member which is sucked towards the holding surface using the suction air from the suction hole, in a predetermined range of a travel path of the holding surface, the belt member being arranged opposing the holding surface along the travel path and being arranged movably along the travel path over the predetermined range of the travel path, and being arranged to sandwich the absorbent body between the holding surface and the belt member.

Other features of this invention will become clear from the present specification and the description of the attached drawings.

### Advantageous Effects of Invention

According to this invention, a thickness of an absorbent body can be made thin, by suppressing excessive squashing of superabsorbent polymer.

### Brief Description of the Drawings

Fig. 1 is a central vertical cross sectional view of an example of a manufacturing apparatus 10 used in a manufacturing method of an absorbent body 1.
Fig. 2A and Fig. 2B are explanatory views of a thinning process of the absorbent body 1 according to a first embodiment;
Fig. 3 is a III-III cross sectional view of Fig. 1.
Fig. 4 is a central vertical cross sectional view of the absorbent body 1 according to a modified example of the first embodiment a manufacturing apparatus 10b.
Fig. 5 is a perspective view of a rotating drum 20g of a fiber stacking apparatus 11b.
Fig. 6 is an explanatory view of a manufacturing method of an absorbent body 1 to which a thinning process of a second embodiment has been applied, and shows a central vertical cross sectional view of the manufacturing apparatus 10c.
Fig. 7 is an enlarged view of a thinning apparatus 61 according to a second embodiment.
Fig. 8 is a VIII-VIII cross sectional view of Fig. 7.
Fig. 9 is an explanatory view of an example of zone division of a first zone Z11.

### DESCRIPTION OF EMBODIMENTS

At least the following matters will become clear through the description of the present specification and the accompanying drawings.

A method which makes a thickness of an absorbent body, including a liquid absorbent fiber and a superabsorbent polymer, thin, the method comprising:
transporting the absorbent body by moving a holding surface while holding the absorbent body on the holding surface with suction air from a suction hole on the holding surface; and
making the thickness of the absorbent body thin by
   sucking a belt member towards the holding surface using the suction air from the suction hole, in a predetermined range of a travel path of the holding surface, the belt member being arranged opposing the holding surface along the travel path and being arranged movably along the travel path over the predetermined range of the travel path, and
   sandwiching the absorbent body between the holding surface and the belt member.

According to this method, which makes a thickness of an absorbent body thin, a member which sandwiches the absorbent body in association with a holding surface is a belt member that has been movably arranged along the travel path over a predetermined range of the travel path of the holding surface. Therefore, in the predetermined range, the belt member can sandwich and press the absorbent body in a surface contacting state, in association with the holding surface, and therefore, an excessive compression force acting on the superabsorbent polymer can be effectively suppressed. As a result, while suppressing excessive squashing of the superabsorbent polymer in the absorbent body, the thickness of the absorbent body can be made thin.

Further, the sandwiching and pressing force when sandwiching the absorbent body occurs based on the suction power that acts on the belt member by the suction air. Therefore, compared to hydraulic pressure and the like, the absorbent body can be softly sandwiched, and thus, excessive squashing of the superabsorbent polymer in the absorbent body can be suppressed.

Further, the contact surface of the belt member for contacting the absorbent body, based on the flexibility of the belt member, can follow the concave and convex form of the absorbent body surface due to the presence of the superabsorbent polymer and the like, and thus, excessive squashing of the superabsorbent polymer in the absorbent body can be suppressed.

Further, the absorbent body is maintained in a sandwiched state while passing along the predetermined range. Therefore, the sandwiching and pressing time can be maintained for longer than when sandwiching and pressing using a pair of rollers, and therefore, while softly sandwiching the absorbent body in a surface contacting state, the thickness of the absorbent body can surely be made thin.

Further, since the belt member is movable along the travel path over the predetermined range, in the predetermined range in which the sandwiching process of the absorbent body is performed in association with the holding surface, the belt member can move in the same direction as the holding surface together with the moving holding surface. Therefore, the belt member can become substantially integrated with the holding surface and can suppress any slip between the absorbent body that is being transported and the belt member, and thus, peeling off of the absorbent body from the holding surface can be effectively prevented. As a result, the absorbent body can be stably manufactured.

According to the method which makes the thickness of the absorbent body thin,
the belt member is preferably a rubber belt.

According to this method which makes the thickness of the absorbent body thin, with the flexible elastic deformation of rubber, the contact surface of the belt member for contacting the absorbent body can follow the concave and convex form of the absorbent body surface. Further, similarly with the flexible elastic deformation of rubber, the belt member can suppress squashing of the superabsorbent polymer which is harder than the liquid absorbent fiber and also, of the two, selectively compress the liquid absorbent fiber. Therefore, while effectively avoiding excessive squashing of the superabsorbent polymer in the absorbent body, the thickness of the absorbent body can be made thin.

According to this method which makes the thickness of the absorbent body thin, a hardness of the rubber belt is preferably 35 to 40A in the notation of Shore hardness A scale of ISO (International Organization for Standardization).

According to this method which makes the thickness of the absorbent body thin, since rubber in the above-described hardness range is used, selectively compressing the liquid absorbent fiber rather than the superabsorbent polymer, as described above, can surely be performed.

According to the method which makes the thickness of the absorbent body thin,
the belt member preferably moves in the predetermined range, together with the moving holding surface, with a moving speed of the holding surface as a target speed.

According to this method which makes the thickness of the absorbent body thin, in the predetermined range in which the sandwiching process of the absorbent body is performed in association with the holding surface, the belt member moves at substantially the same speed as the holding surface, together with the moving holding surface. Therefore, the belt member can become substantially integrated with the holding surface and can surely suppress any slip between the absorbent body that is being transported and the belt member. Therefore, peeling off of the absorbent body from the holding surface can be more effectively prevented.

According to the method which makes the thickness of the absorbent body thin, preferably
the holding surface is a portion of a predetermined surface included in a predetermined member,
the predetermined surface has a width direction intersecting a direction along the travel path, and
the belt member closely contacts a portion at an outer side in the width direction of the holding surface of the predetermined surface, in the predetermined range of the travel path.

According to this method which makes the thickness of the absorbent body thin, the belt member closely contacts the portion at the outer side in the width direction of the holding surface on which the absorbent body is being held. Therefore, airtightness of an accommodating space of the absorbent body that is defined by the belt member and the holding surface can be increased. As a result, the air pressure in the space in between the fibers in the absorbent body can be effectively decreased, and therefore the suction power acting on the belt member from the suction holes increases, and thus the sandwiching and pressing force on the absorbent body of the belt member and the holding surface can be increased.

According to the method which makes the thickness of the absorbent body thin, preferably
the absorbent body is molded by a fiber stacking apparatus,
the fiber stacking apparatus has a mold formed in a concave shape on an outer circumferential face, a first rotating drum that continuously rotates in one direction in a circumferential direction, a supply duct provided in a predetermined position in the circumferential direction and that supplies a gas, with the liquid absorbent fiber and superabsorbent polymer mixed therein, towards the outer circumferential face, and a mold-release mechanism that releases the absorbent body from the mold and that is provided on a downstream side of the predetermined position in the circumferential direction,
the holding surface is a bottom section of the mold, and
the belt member is arranged opposing the outer circumferential face, in a position between the supply duct and the mold-release mechanism in the circumferential direction of the first rotating drum.

According to this method which makes the thickness of the absorbent body thin, the belt member is arranged opposed to the outer circumferential face of the first rotating drum, and the process to make the thickness of the absorbent body thin is performed with the first rotating drum. Therefore, it is not necessary to provide other rotating drums and the like, and simplification and space saving of the apparatus configuration can be realized.

According to the method which makes the thickness of the absorbent body thin, preferably
the absorbent body is molded by the fiber stacking apparatus,
the fiber stacking apparatus has a mold formed in a concave shape on an outer circumferential face, a first rotating drum that continuously rotates in one direction in a circumferential direction, and a supply duct provided in a predetermined position in the circumferential direction and that supplies a gas, with the liquid absorbent fiber and the superabsorbent polymer mixed therein, towards the outer circumferential face, and
in making the thickness of the absorbent body thin,
includes a second rotating drum provided on a downstream side of the predetermined position in the circumferential direction, and that receives the absorbent body from the mold of the first rotating drum,
the holding surface is a portion of an outer circumferential face of the second rotating drum, and the second rotating drum rotates in one direction in a circumferential direction, while the holding surface holds the absorbent body received from the first rotating drum on the outer circumferential face, using the suction air from the suction hole on the holding surface, and
the belt member is arranged in a predetermined position in the circumferential direction, while opposing the outer circumferential face of the second rotating drum.

According to this method which makes the thickness of the absorbent body thin, the second rotating drum can be used as an apparatus dedicated to making the thickness of the absorbent body thin. Therefore, the specification of the hole diameter of the suction hole and its arrangement pattern and the like on the outer circumferential face of the second rotating drum can be designed to have a specification specialized to making the thickness thin. As a result, making the absorbent body thin, while suppressing squashing of the superabsorbent polymer, can be more surely performed.

According to the method which makes the thickness of the absorbent body thin, preferably
the outer circumferential face of the second rotating drum is formed into a substantially smooth surface, without a recessed portion that has a depth deeper than a thickness of the absorbent body when receiving the absorbent body from the first rotating drum, and
the belt member moves along the outer circumferential face together with the moving holding surface, at least while making the thickness thin, and the belt member is sucked towards the outer circumferential face using the suction air from the suction hole, and sandwiches and presses the absorbent body with the holding surface on the outer circumferential face.

According to this method which makes the thickness of the absorbent body thin, the outer circumferential face of the second rotating drum is formed as a substantially smooth surface, the substantially smooth surface not having a concave portion that is deeper than a thickness of the absorbent body when it is received from the first rotating drum. Therefore, the outer circumferential face can surely sandwich and press the absorbent body protruding from the outer circumferential face with the belt member. As a result, the thickness of the absorbent body can surely be made thin.

According to the method which makes the thickness of the absorbent body thin, preferably
the holding surface is formed on one side of a moving member which moves along the travel path, and on an opposite surface of the holding surface of the moving member is oppositely positioned a space which performs suction of air from the suction hole on the holding surface,
the space is defined into a plurality of zones along the travel path, and an air pressure of a zone corresponding to the predetermined range of the plurality of zones is lower than that of a zone corresponding to a position on a downstream side of the predetermined range, and
an air pressure of a zone corresponding to a downstream end in the predetermined range is set to a value between
an air pressure of a zone other than a zone corresponding to the downstream end of the zones corresponding to the predetermined range and
an air pressure of a zone corresponding to a position on a downstream side of the predetermined range.

According to this method which makes the thickness of the absorbent body thin, the zone corresponding to the downstream end effectively functions as a buffer zone when changing air pressure. In other words, a rapid suction air pressure fluctuation which may occur when the holding surface changes from a low air pressure zone (a zone other than a zone corresponding to the downstream end of the zones corresponding to the predetermined range) to a high air pressure zone (a zone corresponding to a position on a downstream side of a predetermined range) can be relieved, and falling off or peeling off of the absorbent body from the holding surface and the like due to the suction air pressure fluctuation can be effectively prevented.

Further, an apparatus which makes a thickness of an absorbent body, including a liquid absorbent fiber and a superabsorbent polymer, thin, the apparatus comprising:
a first apparatus which transports the absorbent body by having a holding surface which moves while holding the absorbent body on the holding surface with suction air from a suction hole on the holding surface; and
a second apparatus which makes the thickness of the absorbent body thin by having a belt member which is sucked towards the holding surface using the suction air from the suction hole, in a predetermined range of a travel path of the holding surface, the belt member being arranged opposing the holding surface along the travel path and being arranged movably along the travel path over the predetermined range of the travel path, and being arranged to sandwich the absorbent body between the holding surface and the belt member.

According to this apparatus which makes the thickness of the absorbent body thin, a similar effect as the above-described method that makes the thickness of the absorbent body thin can be realized.

### ===First embodiment===

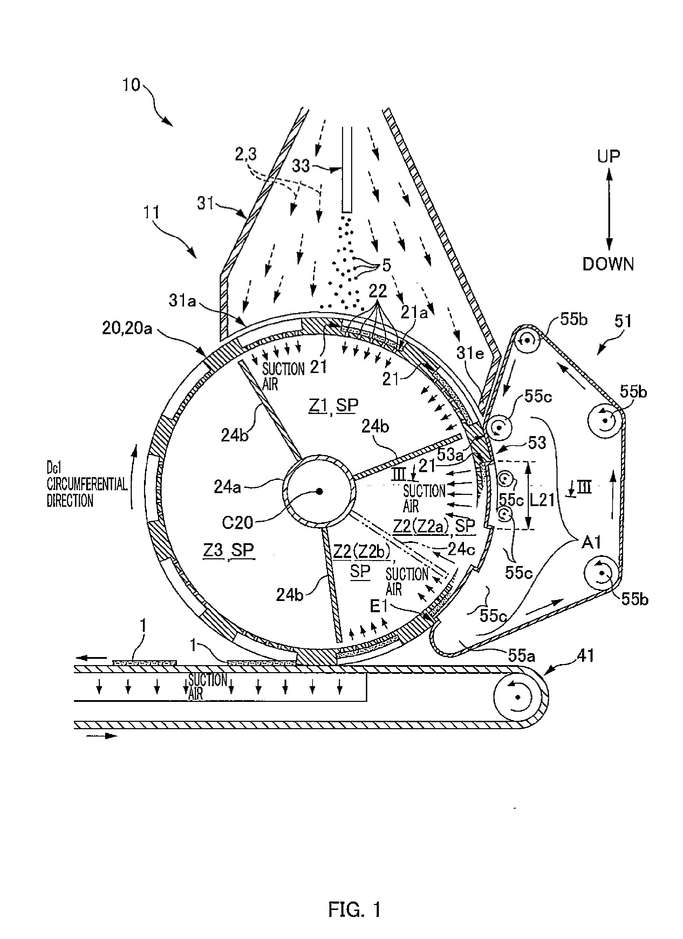

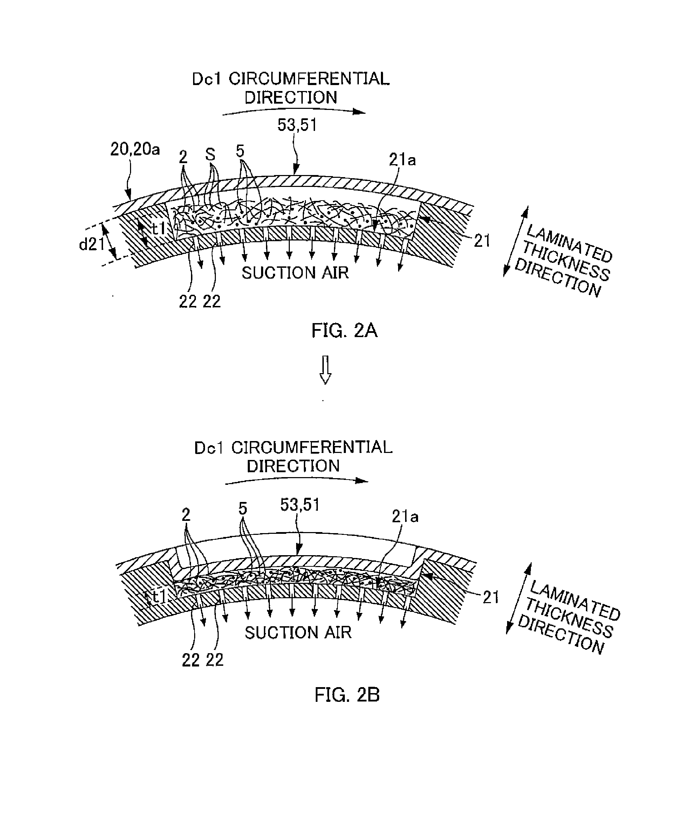

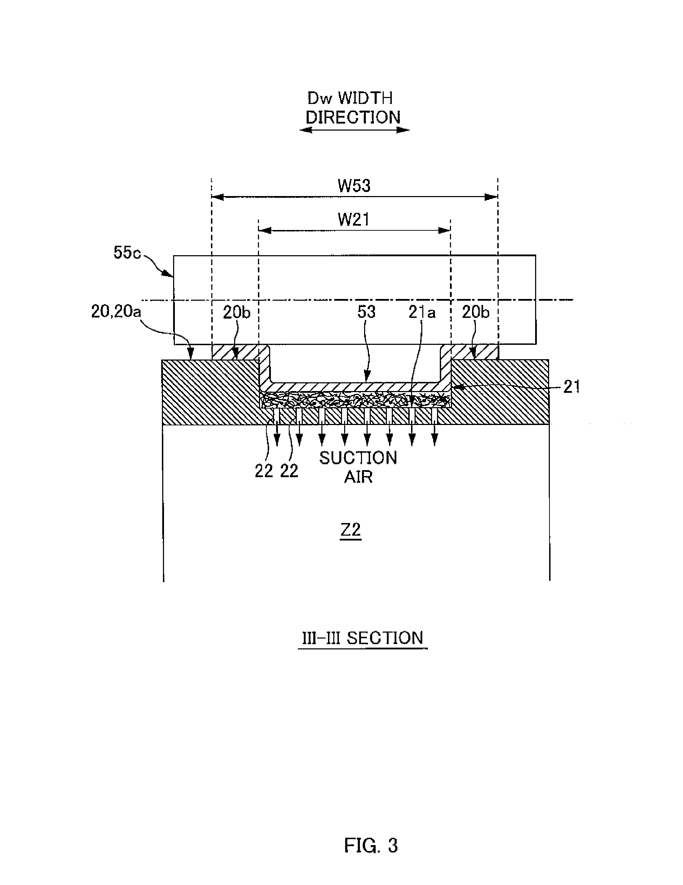

Fig. 1 is a central vertical cross sectional view of an example of a manufacturing apparatus 10 used in a manufacturing method of an absorbent body 1. Fig. 2A and Fig. 2B are explanatory views of a thinning process of the absorbent body 1, and Fig. 2A shows a state before processing or a first half of the processing, and Fig. 2B shows a state in a second half or after processing. Further, Fig. 3 is a III-III cross sectional view of Fig. 1.

A method which makes a thickness of the absorbent body 1 according to a first embodiment, thin is one process in a manufacturing method of this absorbent body 1. In other words, the manufacturing method of the absorbent body 1 includes an absorbent body molding process that molds the absorbent body 1 by laminating pulp fiber 2 (corresponding to liquid absorbent fiber) including superabsorbent polymer 5, and an absorbent body thinning process that makes a lamination thickness of the molded absorbent body 1 thin while substantially maintaining a basis weight, and the method according to this first embodiment is applied in respect of the latter process of these two processes.

The former absorbent body molding process is performed by a so-called fiber stacking apparatus 11, and further, in regards to the latter absorbent body thinning process, a thinning apparatus 51 arranged near the fiber stacking apparatus 11 is to perform the process in association with the fiber stacking apparatus 11. In other words, the fiber stacking apparatus 11 corresponds to a "first apparatus" and a "second apparatus", and the thinning apparatus 51 corresponds to the "second apparatus". Hereinbelow, the fiber stacking apparatus 11 and the thinning apparatus 51 are described.

### <<<Fiber stacking apparatus 11>>>

The fiber stacking apparatus 11 includes, for example, (1) a rotating drum 20 that drivingly rotates continuously in one direction of a circumferential direction Dc1 (for example, clockwise) with a horizontal shaft C20 as a rotational center, (2) a supply duct 31 that discharges and supplies mixed air 3 including pulp fiber 2 toward an outer circumferential face 20a of the rotating drum 20 from a supply opening 31a arranged in a predetermined position in the circumferential direction Dc1 of the rotating drum 20, (3) a polymer inserting duct 33 included in the supply duct 31 and that discharges the granular superabsorbent polymer 5 towards the outer circumferential face 20a, and (4) a suction conveyor 41 (corresponding to a mold-release mechanism) arranged on a downstream side in the circumferential direction Dc1 of the supply duct 31, and that sucks the absorbent body 1 in order to mold-release it from a mold 21 on the outer circumferential face 20a of the rotating drum 20 and transports it.

Hereinbelow, the circumferential direction Dc1 of the rotating drum 20 is merely referred to as the "circumferential direction Dc1", and a direction along the horizontal shaft C20 of the rotating drum 20 (a direction passing through a plane of paper in Fig. 1) is referred to as a "width direction Dw". Note that this width direction Dw is perpendicular to the circumferential direction Dc1.

The rotating drum 20 (corresponding to a moving member, a first rotating drum) is a substantially cylindrical body, and on its outer circumferential face 20a is included, intermittently in a predetermined pitch in a circumferential direction Dc1, the molds 21 in a concave form corresponding to a form of the absorbent body 1 to be molded. Then, at a bottom section 21a of each mold 21 (corresponding to a holding surface) are provided a plurality of suction holes 22, and through these suction holes 22 an inner side of the mold 21 is in communication with an inner circumferential side of the rotating drum 20 in a permeable manner.

On the other hand, on the inner circumferential side of the rotating drum 20 is included a cylindrical division wall 24a concentrically with the rotating drum 20, and thus on the inner circumferential side of the rotating drum 20 is defined a doughnut shaped substantially closed space SP (corresponding to a space). Further, this substantially closed space SP is zone divided in the circumferential direction Dc1 by a plurality of division walls 24b, 24b, 24b. Therefore, for example, a first zone Z1 shown in Fig. 1 is maintained at a negative pressure state with an air pressure lower than the outside air pressure, and on the other hand, an air pressure P3 of a third zone Z3 on a further downstream side thereof is maintained at the same pressure as the outside air pressure or at an air pressure value in between the outside air pressure and an air pressure P1 of the first zone Z1. Then, in correspondence to this first zone Z1, a supply opening 31a of the supply duct 31 is arranged, and on the other hand the suction conveyor 41 is arranged in correspondence to the third zone Z3. Note that, a second zone Z2 positioned in between these first and third zones Z1, Z3 is related to the absorbent body thinning process and this will be described later.

Then, according to such fiber stacking apparatus 11, the absorbent body 1 is molded as follows. Firstly, by the driving rotation of the rotating drum 20, the mold 21 is moved along a path, along the outer circumferential face 20a, as a travel path. Then, when this mold 21 is passing the position of the supply duct 31, substantially only air of the mixed air 3 discharged and supplied from the supply opening 31a is sucked into the suction holes 22 on the bottom section 21a of the mold 21, and thus on the bottom section 21a are laminated the pulp fiber 2 and the superabsorbent polymer 5 in the mixed air 3 and the absorbent body 1 is manufactured. Then, when the mold 21 reaches a position opposing the suction conveyor 41, via a position of the thinning apparatus 51 to be described later, the absorbent body 1 in the mold 21 is sucked toward the outside by the suction air from the suction conveyor 41 and successively mold-released from the mold 21, and thereafter transported by the suction conveyor 41.

### <<<Thinning apparatus 51>>>

The thinning apparatus 51 is to make a lamination thickness of the absorbent body 1 thin, and this apparatus 51 is arranged in a position between the supply duct 31 and the suction conveyor 41 in the circumferential direction Dc1 of the rotating drum 20 and opposing the outer circumferential face 20a of the rotating drum 20.

This thinning apparatus 51 has a non-air permeable endless belt 53 (corresponding to a belt member) that moves in a predetermined circular orbit as a main body, and this endless belt 53 has a portion of its circular orbit arranged along a portion of the outer circumferential face 20a of the rotating drum 20 in an arc shape. Therefore, in a range A1 along this arc shape (hereinbelow, referred to as a belt setting range A1), the endless belt 53 covers the absorbent body 1 on a side opposite from the bottom section 21a of the mold 21 by its flat outer circumferential face 53a, and moves together with the movement of the mold 21 in the circumferential direction Dc1 at substantially the same speed as the mold 21.

Then, while the mold 21 is moving in this belt setting range A1, air in a space S in between the pulp fibers in the absorbent body 1 is sucked up by the suction air from the suction holes 22 on the bottom section 21a of this mold 21, and at this time, the endless belt 53 effectively suppresses the inflow of outside air into the absorbent body 1 with its non-air permeability. Therefore, an air pressure Pf2 of the space S in between the pulp fibers in the absorbent body 1, that has been laminated and molded in the mold 21, is made lower than an air pressure Pf1 of the space S in between the pulp fibers in the absorbent body 1 at the time of lamination at the above supply duct 31, and therefore, the air in the space S in between the pulp fibers in the absorbent body 1 is effectively sucked up to move the fibers 2 towards the bottom section 21a so that the pulp fibers 2 become thickly packed, from a state shown in Fig. 2A to a state shown in Fig. 2B, and the lamination thickness t1 of the absorbent body 1 is made thin.

Further, concurrently with the above, as shown in Fig. 2B, the endless belt 53 is sucked towards the bottom section 21a of the mold 21 by the suction air from the suction holes 22 and is drawn into the mold 21, and at this time, a portion of the endless belt 53 that has been drawn in sandwiches the absorbent body 1 with the bottom section 21a, and therefore the lamination thickness of the absorbent body 1 is made thinner. This sandwiching and pressing is performed by the endless belt 53 surface contacting substantially evenly with the absorbent body 1, and thus excessive compression force acting on the superabsorbent polymer 5 in the absorbent body 1 is effectively suppressed. Therefore the thickness of the absorbent body 1 can be made thin while preventing the superabsorbent polymer 5 from being excessively squashed.

Further, this sandwiching and pressing force is based on the suction power that acts on the endless belt 53 using the suction air. Therefore, compared to hydraulic pressure and the like, the absorbent body 1 can be softly sandwiched, and thus the superabsorbent polymer 5 in the absorbent body 1 can be suppressed from being excessively squashed.

Further, the contact surface of the endless belt 53 to the absorbent body 1 elastically deforms rapidly based on the flexibility of the endless belt 53, and therefore can follow a concave-convex form of the absorbent body 1 surface due to the presence of the superabsorbent polymer 5. In other words, due to the flexible elastic deformation of the endless belt 53, the superabsorbent polymer 5 that is harder than the pulp fiber 2 can be suppressed from being squashed and, of the two, the pulp fiber 2 can be selectively compressed. Therefore, in this way, the superabsorbent polymer 5 in the absorbent body 1 can be suppressed from being excessively squashed. Note that, in order for this effect to be more surely exerted, preferably, the endless belt 53 is made of rubber that can elastically deform flexibly, and more preferably the hardness of the rubber is 35 to 40A in the notation of Shore hardness A scale of ISO (International Organization for Standardization).

Further, according to the above configuration, while the mold 21 is passing through the belt setting range A1, a state where the absorbent body 1 is sandwiched by the endless belt 53 and the bottom section 21a is maintained. Therefore, a sandwiching and pressing time can be secured for a long time, and thus the absorbent body 1 is softly sandwiched in a surface contact state, and a lack of sandwiching and pressing force due to the soft sandwiching and pressing can be compensated for by the sandwiching and pressing time, and as a result the thickness of the absorbent body 1 can surely be made thin.

Further, in cooperation with the bottom section 21a of the mold 21, in the belt setting range A1 in which the endless belt 53 is sandwiching the absorbent body 1, the endless belt 53 moves together with the movement of the mold 21 at substantially the same speed. Therefore, any slip between the absorbent body 1, that is being transported substantially integrally with the bottom section 21a of the mold 21, and the endless belt 53 can be suppressed, and therefore peeling off of the absorbent body 1 from the mold 21 can be prevented. As a result, the absorbent body 1 can be stably manufactured.

By the way, suction of air of the suction holes 22 in this belt setting range A1 is performed based on an air pressure P2 of the second zone Z2 in the rotating drum 20. Then, a value of the air pressure P2 of the second zone Z2 is set as, for example, an air pressure value where an air pressure Pf2 of the space S in between the pulp fibers in the absorbent body 1 in a state where the mold 21 is covered by the endless belt 53 becomes lower than an air pressure Pf1 of the space S in between the pulp fibers in the absorbent body 1 at the time of lamination by the supply duct 31, and moreover as an air pressure value where a suction power level that draws the endless belt 53 toward the absorbent body 1 until the endless belt 53 surface contacts the absorbent body 1 can be achieved.

As an example, the air pressure P2 of the second zone Z2 is set as the same pressure or a lower pressure than the air pressure P1 of the first zone. The reason that it can be the same pressure is because when laminating in the first zone Z1, the mixed air 3 is supplied into the supply duct 31, but in the second zone Z2, the mixed air 3 is not supplied, and inflow of the outside air into the absorbent body 1 can be suppressed by the endless belt 53.

Note that, in some cases, the second zone Z2 may be further subdivided into a plurality of zones in the circumferential direction Dc1 by a division wall 24c. In the example in Fig. 1, it is subdivided into two zones Z2a, Z2b. Then, in the case of such subdivision, preferably, an air pressure P2b of a zone Z2b corresponding to a downstream end E1 in the circumferential direction Dc1 of the belt setting range A1 may be set to a value in between the air pressure P2a of the zone Z2a adjacent to the upstream side thereof and an air pressure P3 of the third zone Z3 adjacent to the downstream side thereof. Then, the zone Z2b corresponding to the downstream end E1 of the belt setting range A1 effectively functions as a buffer zone in air pressure change, in other words, a sudden air pressure fluctuation that may occur when the mold 21 moves from the low air pressure second zone Z2 to the high air pressure third zone Z3 can be relieved, and dropping off or peeling off and the like of the absorbent body 1 from the mold 21 due to the suction air pressure fluctuation can be effectively prevented.

Here, preferably, from the view of inflow suppression of outside air into the absorbent body 1, preferably a width W53 of the endless belt 53 is made wider than a size W21 of the width direction Dw of the mold 21 as shown in Fig. 3. Then, the endless belt 53, that has been sucked towards the outer circumferential face 20a by the suction air from the suction holes 22 of the mold 21, adopts a closely contacting state for each portion 20b, 20b at an outer side in the width direction Dw of the mold 21 on the outer circumferential face 20a (corresponding to a predetermined surface) of the rotating drum 20 (corresponding to a predetermined member) as shown in Fig. 3. Therefore, inflow of the outside air from the width direction Dw is effectively prevented. Further, from a similar view, in addition to the above, preferably a length in the circumferential direction Dc1 of the belt setting range A1 shown in Fig. 1 is set longer than an entire length L21 of the circumferential direction Dc1 of the mold 21, and more preferably, set longer than 1.5 times of the entire length L21. Then, the mold 21 in which the air in the space S in between the pulp fibers is being sucked up can be covered up by the endless belt 53 from all directions (all directions of the width direction Dw and the circumferential direction Dc1) of its four peripheral edges, and therefore outside air inflow into the absorbent body 1 can be almost completely prevented.

Further, as can be understood by referring to Fig. 2A, a depth direction and a lamination thickness direction of the mold 21 are in the same direction. Therefore, preferably, a depth d21 of the bottom section 21a of the mold 21, with the outer circumferential face 20a of the rotating drum 20 as a reference, is set in a range of 0.5 times to 1.0 times a target lamination thickness t1 of the absorbent body 1 when laminating by the supply duct 31. Then, in the process of the thinning apparatus 51, the absorbent body 1 can be pressed by the endless belt 53, without the endless belt 53 greatly getting into a deep side in the mold 21. Therefore, the lamination thickness of the absorbent body 1 can be made more surely thin.

By the way, as shown in Fig. 1, a circular orbit of the endless belt 53 is formed by the endless belt 53 being put around a plurality of pass-line rollers 55a, 55b, 55c arranged in predetermined positions. Then, at least one of these pass-line rollers 55a, 55b, 55c is configured as a driving roller 55a connected to a driving source such as a motor. Therefore, by appropriately speed-controlling and the like the driving source, and drive-controlling the moving speed of the endless belt 53 in the belt setting range A1, with the moving speed of the mold 21 as a target speed, then the endless belt 53 and the mold 21 can be moved at substantially the same speed as each other as described above.

Here preferably, of these pass-line rollers 55a, 55b, 55c, the rollers 55c, 55c ... that are in charge of an orbit of the belt setting range A1, in other words, the rollers 55c, 55c ... that are in charge of an arc shaped orbit along the outer circumferential face 20a of the rotating drum 20, are guided movably in a separating direction in respect to the outer circumferential face 20a of the rotating drum 20 by appropriate guide members, and a pressing force is added in a pressing direction to the outer circumferential face 20a by an elastic member such as a spring. Then, the endless belt 53 can be easily maintained in a surface contacting state to the outer circumferential face 20a of the rotating drum 20, and the airtightness in the mold 21 is increased (refer to Fig. 3). Further, a tension of the endless belt 53 can be easily maintained at a certain value, so that a running state of the endless belt 53 can also be stabilized.

Further, from the view of suppression of entry of the outside air into the supply duct 31, preferably as shown in Fig. 1, the circular orbit of the endless belt 53 may be set so that an end edge 31e at a downstream side in the circumferential direction Dc1 of the supply opening 31a of the supply duct 31 is covered over in the circumferential direction Dc1 by the endless belt 53. In this way, the outside air that enters into the supply duct 31 from a gap between the end edge 31e of the supply opening 31a and the outer circumferential face 20a of the rotating drum 20 can be suppressed, and lamination by the supply duct 31 can be stabilized.

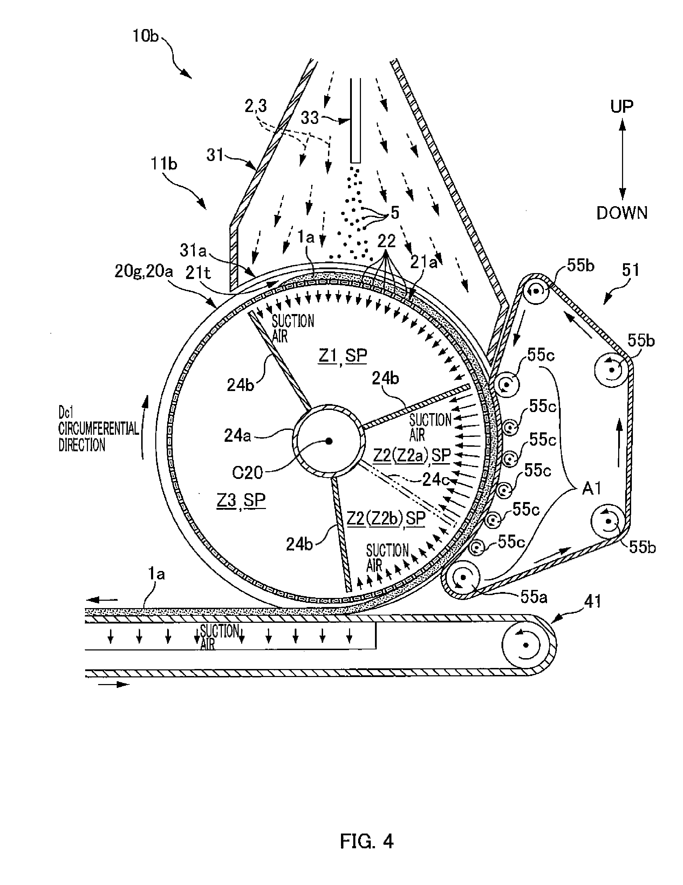

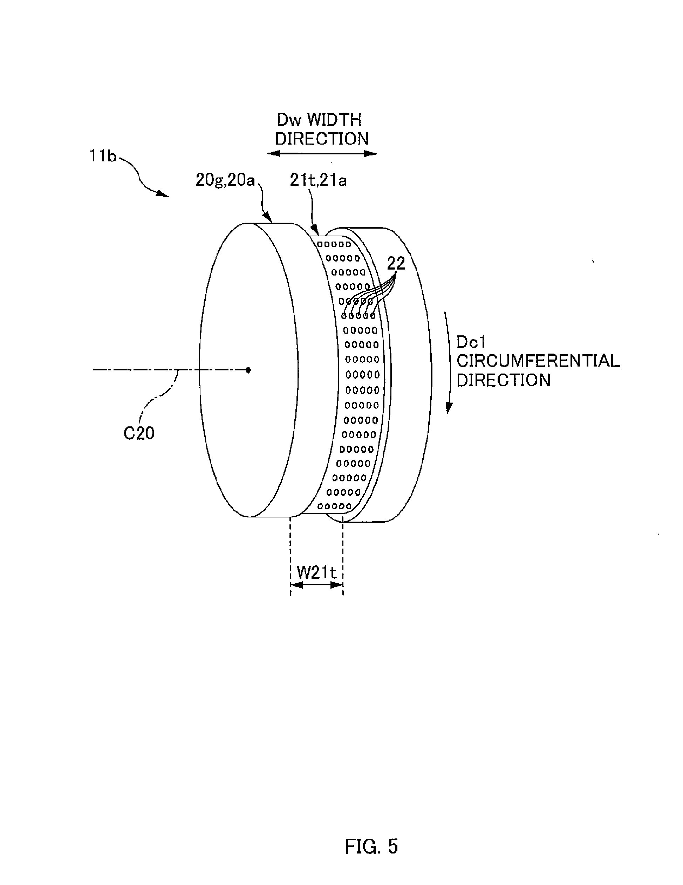

Fig. 4 and Fig. 5 are explanatory views of a modified example of the first embodiment. Fig. 4 is a central vertical cross sectional view of a manufacturing apparatus 10b of the absorbent body 1. Fig. 5 is a perspective view of a rotating drum 20g of a fiber stacking apparatus 11b.

In the first embodiment, the absorbent body 1 is intermittently molded in the circumferential direction Dc1 of the rotating drum 20, but this modified example differs in that a continuous body 1a of the absorbent body is molded, the continuous body 1a of the absorbent body being made with the absorbent body 1 continuously in the circumferential direction Dc1. In other words, as shown in Fig. 5, on the outer circumferential face 20a of the rotating drum 20g of the fiber stacking apparatus 11b according to this modified example, a single groove portion 21t that continues endlessly along the circumferential direction Dc1 is formed as the mold 21t. Then, at a bottom section 21a of this groove portion 21t, as shown in Fig. 4, a plurality of suction holes 22 is provided over the entire circumference of the circumferential direction Dc1, and the pulp fiber 2 and the like are laminated in the groove portion 21t with suction air from these suction holes 22, and thus the continuous body 1a of the absorbent body is molded. Other parts are substantially the same as the above first embodiment.

Here, in the case of this modified example, preferably, a width W53 of the endless belt 53 according to the above thinning apparatus 51 is made thinner than a width W21t of the groove portion 21t. Then, the endless belt 53 that has been sucked toward the groove portion 21t by the suction air from the suction holes 22 of the groove portion 21t can speedily enter into the groove portion 21t, and thus the continuous body 1a of the absorbent body is surely compressed in the lamination thickness direction by a pressing force from the endless belt 53, and as a result the lamination thickness of the continuous body 1a can be made thinner.

Incidentally, in the case where a width W53 of the endless belt 53 is made thinner than a width W21t of the groove portion 21t, a portion of the continuous body 1a of the absorbent body that cannot be covered by the endless belt 53 becomes large, and a suppressing effect of the outside air inflow into the continuous body 1a of the absorbent body decreases. Therefore, the width W53 of the endless belt 53 is preferably made thin in a range of 0.4 times to 0.8 times of the width W21t of the groove portion 21t.

Further, preferably, in respect to the rollers 55c, 55c ... that are in charge of the orbit of the belt setting range A1, the width size may be made thin in a range of 0.5 times to 0.8 times of the width 21t of the groove portion 21t. Then, as shown in Fig. 4, these rollers 55c, 55c ... can quickly enter into the deep side in the groove portion 21t, and thus these rollers 55c, 55c ... can also greatly contribute to pressing with force the absorbent body 1 via the endless belt 53, and as a result the thinning ability is improved.

### === Second embodiment ===

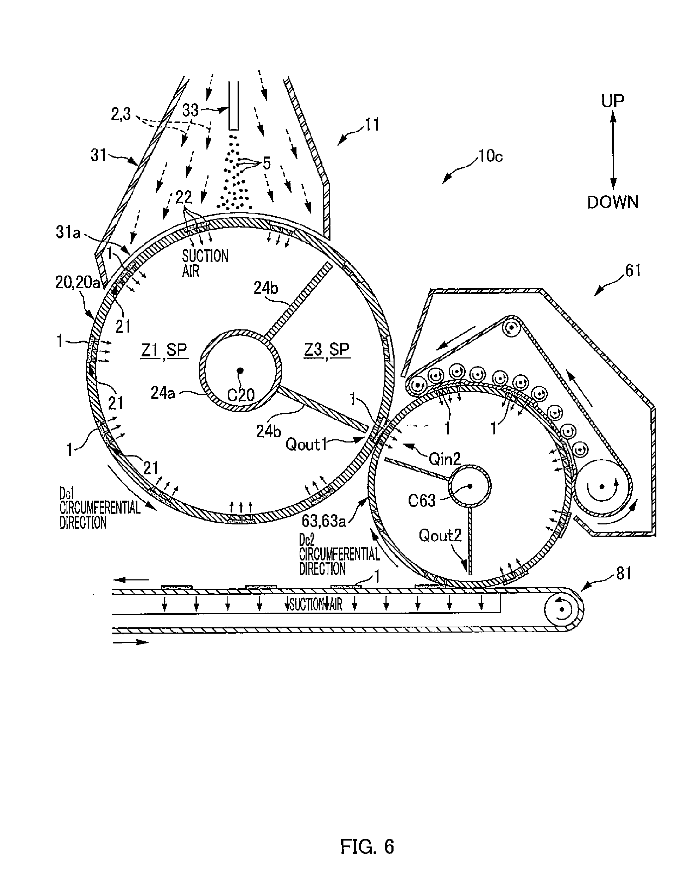

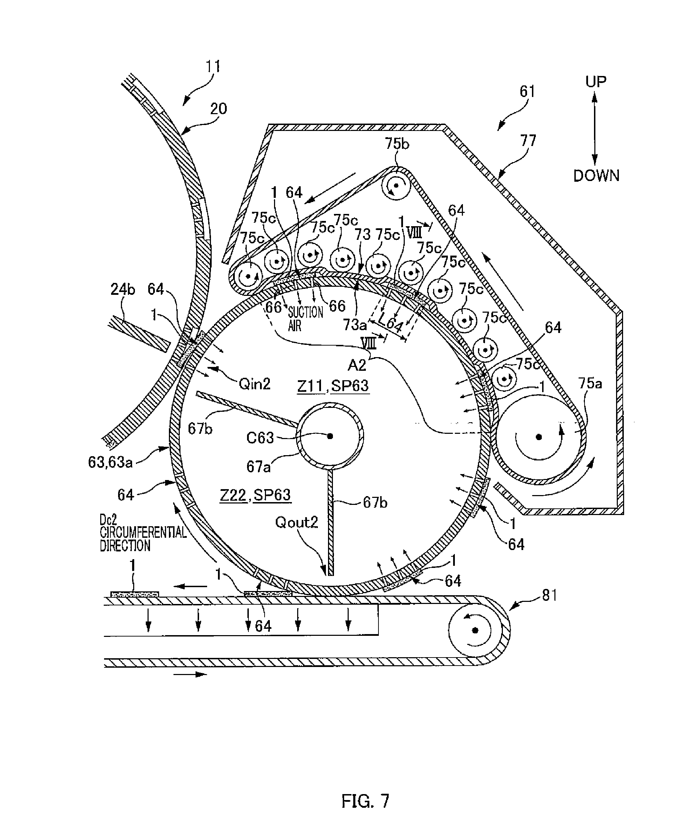

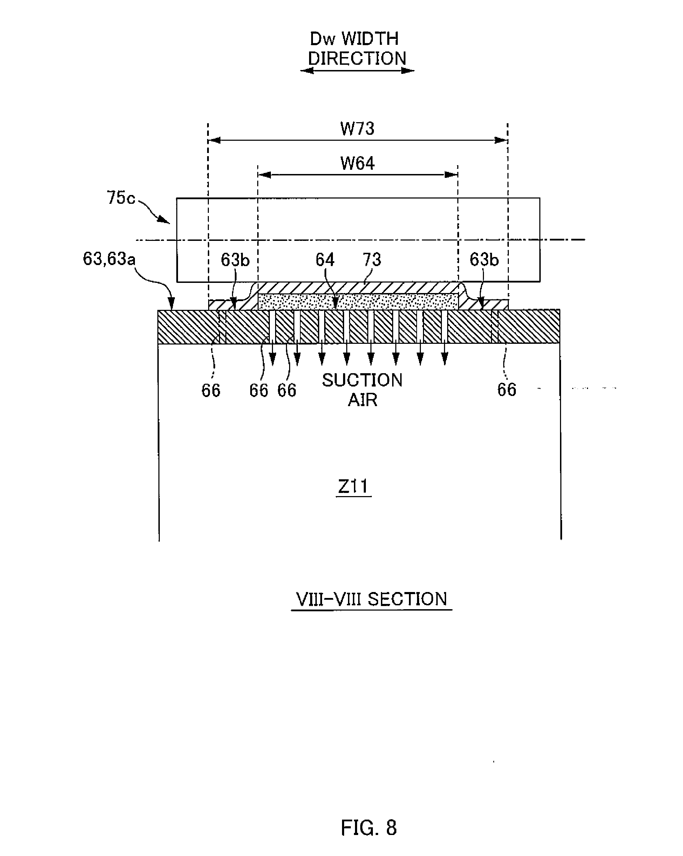

Fig. 6 is an explanatory view of a manufacturing method of the absorbent body 1 which has been applied with a thinning process of a second embodiment, and in other words shows a central vertical cross sectional view of such manufacturing apparatus 10c. Fig. 7 is an enlarged view of a thinning apparatus 61 equipped in this manufacturing apparatus 10c. Further, Fig. 8 is a VIII-VIII cross sectional view of Fig. 7.

In the first embodiment, the fiber stacking apparatus 11 bears a part of the absorbent body thinning process, but in this second embodiment, the fiber stacking apparatus 11 performs merely only the absorbent body molding process, and is generally not engaged in the absorbent body thinning process. In other words, the thinning apparatus 61 according to the second embodiment receives the absorbent body 1 molded by the fiber stacking apparatus 11, and thereafter, the thinning apparatus 61 makes the lamination thickness of the absorbent body 1 thin, while transporting the absorbent body 1. In other words, the fiber stacking apparatus 11 corresponds to the "first apparatus", and the thinning apparatus 61 corresponds to the "second apparatus".

Then, according to this functionally differentiated configuration, the transferring drum 63 to be described later according to the thinning apparatus 61 can be used as an apparatus dedicated to making the lamination thickness of the absorbent body 1 thin. In other words, there is an advantage that the specification of a hole diameter of the suction holes 66, to be described later, on the outer circumferential face 63a of the transferring drum 63 and its arrangement pattern arrangement and the like can be designed as specifications specialized in making the lamination thickness thin.

The fiber stacking apparatus 11 is a generally similar structure as in the first embodiment. In other words, the fiber stacking apparatus 11 has a rotating drum 20 that drivingly rotates in one direction of the circumferential direction Dc1 (for example anticlockwise) with a horizontal shaft C20 as a rotational center, and is arranged with a supply duct 31 in a predetermined position in the circumferential direction Dc1 of the rotating drum 20. Further, in a position on a downstream side in the circumferential direction Dc1 of the supply duct 31, a delivery position Qout1 for handing over the absorbent body 1 that has been molded in the mold 21 on the outer circumferential face 20a of the rotating drum 20 by the supply duct 31 to the thinning apparatus 61 is set. Note that, configurations that are the same as or similar to those in the first embodiment are denoted with the same figures and explanations are omitted.

The thinning apparatus 61 also has a rotating drum 63 (hereinbelow, referred to as a transferring drum 63 (corresponding to a moving member, a second rotating drum)). This transferring drum 63 is also a substantially cylindrical body, and drivingly rotates in one direction (for example, clockwise) in the circumferential direction Dc2 together with the rotating drum 20, with a shaft C63, that is parallel with the shaft C20 of the rotating drum 20 of the fiber stacking apparatus 11, as a rotational center. Further, this transferring drum 63 is arranged, at the above delivery position Qout1, with its outer circumferential face 63a opposing the outer circumferential face 20a of the rotating drum 20 of the fiber stacking apparatus 11. Therefore, on the transferring drum 63 side, with the delivery position Qout1 at a receiving position Qin2, the absorbent body 1 on the outer circumferential face 20a of the rotating drum 20 is received on the outer circumferential face 63a of the transferring drum 63 and held on the outer circumferential face 63a. Then, with the driving rotation of the transferring drum 63, while transporting the absorbent body 1 in the circumferential direction Dc2 along a path, along its outer circumferential face 63a, as a travel path, the absorbent body 1 is subject to a process to make its lamination thickness thin, in other words a thinning process, and thereafter, the absorbent body 1 is transported to the delivery position Qout2 in the circumferential direction Dc2, handed over to the suction conveyor 81 at the same position Qout2, and thereafter transported by the suction conveyor 81.

As shown in an enlarged view of Fig. 7, the outer circumferential face 63a of the transferring drum 63 is formed as a substantially smooth surface, and on this outer circumferential face 63a is set intermittently in a predetermined pitch in the circumferential direction Dc2 a plurality of holding surfaces 64 to hold the received absorbent body 1. Each holding surface 64, respectively, is a smooth surface with an area corresponding to a planar size of the absorbent body 1, and each holding surface 64 is arranged with a plurality of the suction holes 66 in an appropriate arrangement pattern such as a grid arrangement or a staggered arrangement. Then, each holding surface 64 has an ability to retain the absorbent body 1 using the suction air from these suction holes 66.

A suction air source of these suction holes 66 is a negative pressure space Z11 at the inner circumferential side of the transferring drum 63. More specifically, on the inner circumferential side of the transferring drum 63 is included a cylindrical division wall 67a concentrically with the transferring drum 63, and thus, on the inner circumferential side of the transferring drum 63 is defined a doughnut shaped substantially closed space SP63 (corresponding to a space). Further, this substantially closed space SP63 is zone divided in the circumferential direction Dc2 by a plurality of division walls 67b, 67b.

For example, the space is divided into two to form a first zone Z11 from a receiving position Qin2 to a delivery position Qout2, and a second zone Z22 from a delivery position Qout2 to a receiving position Qin2. Then, the first zone Z11 is maintained in a negative pressure state at an air pressure lower than the outside air pressure, and thus, by the suction air from the suction holes 66 on the holding surface 64, the absorbent body 1 received at the receiving position Qin2 is adsorbed and held on the holding surface 64. On the other hand, the second zone Z22 is maintained at an air pressure that is the same or slightly higher than the outside air pressure, and thus, handing over of the absorbent body 1 to the suction conveyor 81 at the delivery position Qout2 can be performed smoothly.

Here, an area of the thinning process to make the absorbent body 1 thin in the circumferential direction Dc2 of the transferring drum 63 is set in a range corresponding to the first zone Z11. In other words, in a portion of this range is arranged a non-air permeable rubber endless belt 73 (corresponding to a belt member) that moves in a predetermined circular orbit. More specifically, this endless belt 73 is arranged with a portion of its circular orbit in an arc shape along the outer circumferential face 63a of the transferring drum 63. Therefore, in the range (hereinbelow, referred to as a belt setting range A2) along this arc shape, the endless belt 73 covers the absorbent body 1 from a side opposite to the holding surface 64 of the transferring drum 63 with its even outer circumferential face 73a and moves at substantially the same speed as the holding surface 64 in the circumferential direction Dc2 together with the movement of the holding surface 64.

Then, while the holding surface 64 is moving in this belt setting range A2, air in a space S in between the pulp fibers in the absorbent body 1 is sucked up by the suction air from the suction holes 66 on the holding surface 64, and at this time, the endless belt 73 effectively suppresses inflow of the outside air into the absorbent body 1. Therefore, an air pressure Pf4 of the space S in between the pulp fibers in the absorbent body 1 that is held on the holding surface 64 can be surely lower than an air pressure Pf1 of the space S in between the pulp fibers in the absorbent body 1 when laminating by the fiber stacking apparatus 11, and as a result the lamination thickness of the absorbent body 1 can be made thin.

Further, as shown in Fig. 7 and Fig. 8, the outer circumferential face 63a of this transferring drum 63 is formed as a substantially smooth surface without a concave portion deeper than the lamination thickness t1 of the absorbent body 1 at the time it is received from the fiber stacking apparatus 11, and therefore the absorbent body 1 is held on the holding surface 64 in a state protruding from the outer circumferential face 63a. Therefore, the endless belt 73, that has been sucked toward the outer circumferential face 63a by the suction air from the suction holes 66 on the holding surface 64, can sandwich and press the absorbent body 1 with the holding surface 64 on the outer circumferential face 63a of the transferring drum 63, without a large concave deformation, as compared to the case of the above-described first embodiment (refer to Fig. 2B and Fig. 3), and therefore the absorbent body 1 can be made thin without applying an unreasonable load that causes deformation on the endless belt 53. Further, at the time of this sandwiching and pressing, the endless belt 73, with its flexible elastic deformation due to it being manufactured by rubber, can suppress squashing of the superabsorbent polymer 5 that is harder than the pulp fiber 2 and, of the two, selectively compress the pulp fibers 2. Note that, in order to surely enjoy these effects, the rubber endless belt 73 with a hardness of 35 to 40A in the notation of Shore hardness A scale of ISO (International Organization for Standardization) is preferably used, as described above.

Needless to say, although a detailed explanation is omitted, the effect of "pressing with force the absorbent body 1 with the endless belt 53 in the surface contacting state", the effect of "the endless belt 53 moving together with the mold 21", and the effect of "the endless belt 53 exerting a sandwiching and pressing force by the suction power based on the suction air" and the like described in the first embodiment may be performed in the same way by the endless belt 73 according to this second embodiment.

By the way, suction of air from the suction holes 66 in this belt setting range A2, is performed based on an air pressure P11 of the above-described first zone Z11. Then, a value of the air pressure P11 of the first zone Z11 is set, for example, so that an air pressure Pf4 in the space S in between the pulp fibers in the absorbent body 1 in a state where the endless belt 73 is covering the absorbent body 1 on the holding surface 64 is decided as an air pressure value that is lower than an air pressure Pf1 of the space S in between the pulp fibers in the absorbent body 1 at the time of lamination by the fiber stacking apparatus 11.

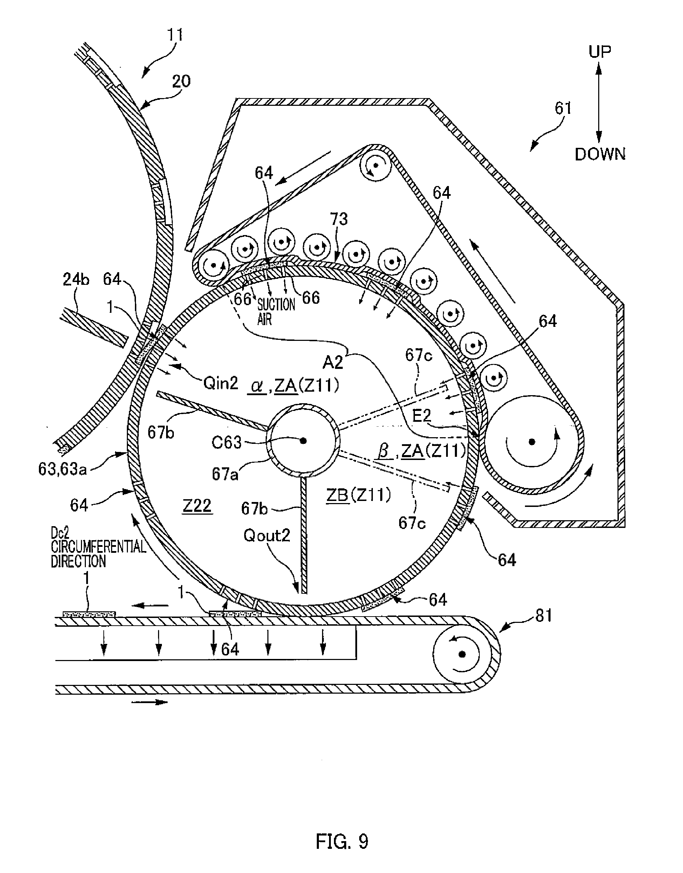

Further, in some cases, the first zone Z11 may be further divided into a plurality of zones in the circumferential direction Dc2 by division walls 67c. For example, in the example of Fig. 9, it is divided into three zones, α, β, and ZB. In other words, firstly, it is divided into an upstream zone ZA, substantially corresponding to the belt setting range A2, and a downstream zone ZB on a downstream side in the circumferential direction Dc2, and further, in respect to the former upstream zone ZA, it is divided into a zone α in its circumferential direction Dc2 and a zone β in its downstream side. Then, in the case of such division in this way, further, in the case where an air pressure Pb in the downstream zone ZB is set higher than an air pressure Pa in the upstream zone ZA, preferably, the air pressure Pβ of the zone β is set to an air pressure value in between the air pressure Pα of the zone α and the air pressure Pb of the downstream zone ZB. Then, the zone β corresponding to a downstream end E2 in the belt setting range A2 functions as a buffer zone in air pressure change, in other words, a rapid suction air pressure fluctuation that may occur when the holding surface 64 moves from the low air pressure upstream zone ZA to the high air pressure downstream zone ZB can be relieved, and falling off or peeling off of the absorbent body 1 from the holding surface 64 due to the suction air pressure fluctuation can be effectively prevented.

Further, preferably, from the view of inflow suppression of the outside air into the absorbent body 1, preferably a width W73 of the endless belt 73 is made wider than a size W64 in the width direction Dw of the holding surface 64 as shown in Fig. 8. Then, the endless belt 73 that has been sucked towards the outer circumferential face 63a by the suction air from the suction holes 66 on the holding surface 64, adopts a closely contacting state for each portion 63b, 63b at the outer side in the width direction Dw of the holding surface 64 on the outer circumferential face 63a (corresponding to a predetermined surface) of the transferring drum 63 (corresponding to a predetermined member) as shown in Fig. 8, and therefore, inflow of the outside air from the width direction Dw is effectively prevented. Further, from a similar view, in addition to the above, preferably a length in the circumferential direction Dc2 of the belt setting range A2 is set longer than an entire length L64 in the circumferential direction Dc2 of the holding surface 64 as shown in Fig. 7. Thus, the holding surface 64 which is sucking up the air in the space S in between the pulp fibers can be covered by the endless belt 73 from all directions of its four peripheral edges (all directions of the width direction Dw and the circumferential direction Dc2), and therefore inflow of the outside air into the absorbent body 1 can be completely prevented. Note that, the suction holes 66 may be formed in respect of each portion 63b, 63b on the outer side of the outer circumferential face 63a (referred to as a chain double-dashed line), and in this way the degree of close contact of the endless belt 73 and each portion 63b, 63b on the outer side can be further increased.

By the way, in the example in Fig. 7, the circular orbit of the endless belt 73 is formed by putting the endless belt 73 around a plurality of pass-line rollers 75a, 75b, 75c arranged in predetermined positions, and at least one of these pass-line rollers 75a, 75b, 75c is configured as a driving roller 75a that has been connected to a driving source such as a motor. Therefore, by appropriate speed controlling and the like of the driving source, with the moving speed of the holding surface 64 as a target speed, when the moving speed of the endless belt 73 in the belt setting range A2 is drive controlled, as described above, the endless belt 73 and the holding surface 64 can be moved at substantially the same speed as each other.

Here, preferably, of these pass-line rollers 75a, 75b, 75c, in respect of the rollers 75c, 75c ... that are in charge of the orbit in the belt setting range A2, in other words, the rollers 75c, 75c ... that are in charge of an arc shaped orbit along the outer circumferential face 63a of the transferring drum 63, are guided movably in a separating direction in respect to the outer circumferential face 63a of the transferring drum 63 by appropriate guide members, and a pressing force is added in a pressing direction to the outer circumferential face 63a by an elastic member such as a spring. Then, with the pressing force, the endless belt 73 is pressed against the outer circumferential face 63a of the transferring drum 63, so that airtightness in the absorbent body 1 and a pressing force on the absorbent body 1 can be increased.

Further, as shown in Fig. 7, the entire circular orbit of this endless belt 73 may be surrounded by a non-air permeable box member 77. Here, the box member 77, in which only a surface opposing the outer circumferential face 63a of the transferring drum 63 is open, is used. Then, in this way, outside air inflow into the absorbent body 1 can be more effectively suppressed.

Further, as the rotating drum 20 of the fiber stacking apparatus 11 according to the above-described second embodiment, the rotating drum 20g according to the modified example of the first embodiment (refer to Fig. 5) may be applied, and thus the lamination thickness in respect to the continuous body 1a of the absorbent body can be made thin.

### === Other Embodiments ===

The embodiments of this invention have been described above, but this invention is not limited to these embodiments and modifications shown hereinbelow are possible.

In the above-described embodiments, as the endless belts 53, 73, those made of rubber are illustrated, but in some cases those made of resin or metal may be used.

In the above-described embodiments, as the belt member, non-air permeable endless belts 53, 73 and the like are illustrated, but they may have slight air permeability. In other words, if it is possible to suck the endless belts 53, 73 toward the bottom section 21a of the mold 21 or the holding surface 64 by the suction air from the suction holes 22, 66, and to sandwich the absorbent body 1 between the bottom section 21a of the holding surface 64 and the belt member, then the belt member may have some air permeability. Needless to say, however, it is preferable that the belt member has no air permeability.

In the above-described first embodiment, as the belt member, the endless belt 53 with a flat outer circumferential face 53a is illustrated, however it is not limited thereto. For example, an endless belt may be used in which at least a portion that is to come into contact with the absorbent body 1 on the outer circumferential face 53a of the endless belt 53, and/or a portion that is to come into contact with the outer circumferential face 20a of the rotating drum 20, is flat, and further an endless belt with the outer circumferential face 53a that is not flat may be used. A flat face is preferable however, because each portion in the outer circumferential face 53a can closely contact with the corresponding portion, and the airtightness in the absorbent body 1 can be increased.

In the above-described second embodiment, as the belt member the endless belt 73 with a flat outer circumferential face 73a is illustrated, but for example, an endless belt may be used in which at least a portion that is to come into contact with the absorbent body 1 on the outer circumferential face 73a of the endless belt 73, and/or a portion that is to come into contact with the outer circumferential face 63a of transferring drum 63, is flat, and further an endless belt with the outer circumferential face 73a that is not flat may be used. A flat face, however, is preferable since each portion in the outer circumferential face 73a closely contacts with the corresponding portion and can increase the airtightness in the absorbent body 1.

In the above-described embodiment, an air permeable continuous sheet such as a tissue paper or a nonwoven fabric was not wrapped around the outer circumferential face 20a of the rotating drums 20, 20g of the fiber stacking apparatuses 11, 11b, but it is not limited thereto. In other words, a continuous sheet may be wrapped around in a predetermined wrap-around angle and the continuous sheet may be transported by rotation of the rotating drums 20, 20g, and when the continuous sheet passes the position on the supply duct 31, the absorbent body 1 may be laminated on the continuous sheet. Note that, in this case, in the second embodiment (Fig. 7), the continuous sheet is transported from the fiber stacking apparatus 11 to the transferring drum 63 integrally with the absorbent body 1, and at the transferring drum 63 the continuous sheet is positioned closer to the endless belt 73 side than the absorbent body 1. Therefore, by the suction air from the suction holes 66 on the outer circumferential face 63a of the transferring drum 63, when the continuous sheet is sucked toward the transferring drum 63, this continuous sheet can press the absorbent body 1, thus the absorbent body 1 can be made further thinner. Further, the continuous sheet is a tissue paper or a nonwoven fabric and the like, and has flexibility, and also functions as a buffer material that relieves the pressing force of the endless belt 73 and transfers it to the absorbent body 1, and thus squashing of the superabsorbent polymer 5 in the absorbent body 1 is suppressed.

In the above-described embodiments, the rotating drums 20, 20g were used as the fiber stacking apparatuses 11, 11b, but it is not limited thereto. For example, the mold 21 may be formed in a concave shape on the belt of the belt conveyor, the suction holes 22 may be included on the bottom section 21a of the mold 21, and the belt may be moved in a predetermined orbit, and the supply duct 31 and the like may be arranged in a predetermined position on that orbit.

Further, similarly, in the above-described second embodiment, as the thinning apparatus 61, the transferring drum 63, in other words the rotating drum 63, was used, but it is not limited thereto. For example, the holding surface 64 may be provided on the surface of the belt of the belt conveyor, and the suction holes 66 may be provided on the holding surface 64, and the belt may be moved in the predetermined orbit, then over the predetermined range on that orbit, the endless belt 73 may be arranged so as to cover the absorbent body 1 that is being held on the holding surface 64.

In the above-described embodiments, air is illustrated as an example of the gas to be discharged and supplied from the supply duct 31, but it is not limited thereto as long as it is a gas that can include, mixed therein, a liquid absorbent fiber such as the pulp fiber 2 and the superabsorbent polymer 5 and that does not chemically react or the like with the fiber and the superabsorbent polymer 5, and it may be a gas such as nitrogen.

In the above-described embodiments, the pulp fiber 2 (a pulp that has been pulverized into a fibrous state) is illustrated as the liquid absorbent fiber, but cellulose such as cotton, regenerated cellulose such as rayon and fibril rayon, semisynthetic cellulose such as acetate and triacetate, fibrous polymer, thermoplastic fiber, or a combination of the above may be used.

### Reference Signs List

1 absorbent body, 1a continuous body of absorbent body (absorbent body), 2 pulp fiber (liquid absorbent fiber), 3 mixed air (gas), 5 superabsorbent polymer, 10 manufacturing apparatus, 10b manufacturing apparatus, 10c manufacturing apparatus, 11 fiber stacking apparatus (first apparatus, second apparatus), 11b fiber stacking apparatus (first apparatus, second apparatus), 20 rotating drum (moving member, first rotating drum, predetermined member), 20a outer circumferential face (predetermined surface), 20b portion, 20g rotating drum (moving member, predetermined member), 21 mold, 21a bottom section (holding surface), 21t groove portion (mold), 22 suction hole, 24a cylindrical division wall, 24b division wall, 24c division wall, 31 supply duct, 31a supply opening, 31e end edge, 33 polymer inserting duct, 41 suction conveyor (mold-release mechanism), 51 thinning apparatus (second apparatus), 53 endless belt (belt member), 53a outer circumferential face, 55a pass-line roller, 55b pass-line roller, 55c pass-line roller, 61 thinning apparatus (second apparatus), 63 transferring drum (moving member, second rotating drum, predetermined member), 63a outer circumferential face (predetermined surface), 63b portion, 64 holding surface, 66 suction hole, 67a cylindrical division wall, 67b division wall, 67c division wall, 73 endless belt (belt member), 73a outer circumferential face, 75a pass-line roller, 75b pass-line roller, 75c pass-line roller, 77 box member, 81 suction conveyor, A1 belt setting region, A2 belt setting region, E1 downstream end, E2 downstream end, SP substantially closed space (space), Z1 first zone, Z2 second zone, Z2a zone, Z2b zone, Z3 third zone, SP63 substantially closed space (space), Z11 first zone, ZA upstream zone, downstream ZB zone, Z22 second zone, Qout1 delivery position, Qin2 receiving position, Qout2 delivery position

## Claims

1. A method which makes a thickness of an absorbent body, including a liquid absorbent fiber and a superabsorbent polymer, thin, the method comprising:
transporting the absorbent body by moving a holding surface while holding the absorbent body on the holding surface with suction air from a suction hole on the holding surface; and
making the thickness of the absorbent body thin by
sucking a belt member towards the holding surface using the suction air from the suction hole, in a predetermined range of a travel path of the holding surface, the belt member being arranged opposing the holding surface along the travel path and being arranged movably along the travel path over the predetermined range of the travel path, and
sandwiching the absorbent body between the holding surface and the belt member.

2. A method which makes a thickness of an absorbent body thin according to claim 1, wherein
the belt member is a rubber belt.

3. A method which makes a thickness of an absorbent body thin according to claim 2, wherein
a hardness of the rubber belt is 35 to 40A in the notation of Shore hardness A scale of ISO (International Organization for Standardization).

4. A method which makes a thickness of an absorbent body thin according to any of claims 1 to 3, wherein
the belt member moves in the predetermined range, together with the moving holding surface, with a moving speed of the holding surface as a target speed.

5. A method which makes a thickness of an absorbent body thin according to any of claims 1 to 4, wherein
the holding surface is a portion of a predetermined surface included in a predetermined member,
the predetermined surface has a width direction intersecting a direction along the travel path, and
the belt member closely contacts a portion at an outer side in the width direction of the holding surface of the predetermined surface, in the predetermined range of the travel path.

6. A method which makes a thickness of an absorbent body thin according to any of claims 1 to 5, wherein
the absorbent body is molded by a fiber stacking apparatus, the fiber stacking apparatus has a mold formed in a concave shape on an outer circumferential face, a first rotating drum that continuously rotates in one direction in a circumferential direction, a supply duct provided in a predetermined position in the circumferential direction and that supplies a gas, with the liquid absorbent fiber and superabsorbent polymer mixed therein, towards the outer circumferential face, and a mold-release mechanism that releases the absorbent body from the mold and that is provided on a downstream side of the predetermined position in the circumferential direction,
the holding surface is a bottom section of the mold, and
the belt member is arranged opposing the outer circumferential face, in a position between the supply duct and the mold-release mechanism in the circumferential direction of the first rotating drum.

7. A method which makes a thickness of an absorbent body thin according to any of claims 1 to 5, wherein
the absorbent body is molded by the fiber stacking apparatus,
the fiber stacking apparatus has a mold formed in a concave shape on an outer circumferential face, a first rotating drum that continuously rotates in one direction in a circumferential direction, and a supply duct provided in a predetermined position in the circumferential direction and that supplies a gas, with the liquid absorbent fiber and the superabsorbent polymer mixed therein, towards the outer circumferential face, and
in making the thickness of the absorbent body thin,
includes a second rotating drum provided on a downstream side of the predetermined position in the circumferential direction, and that receives the absorbent body from the mold of the first rotating drum,
the holding surface is a portion of an outer circumferential face of the second rotating drum, and the second rotating drum rotates in one direction in a circumferential direction, while the holding surface holds the absorbent body received from the first rotating drum on the outer circumferential face, using the suction air from the suction hole on the holding surface, and
the belt member is arranged in a predetermined position in the circumferential direction, while opposing the outer circumferential face of the second rotating drum.

8. A method which makes a thickness of an absorbent body thin according to claim 7, wherein
the outer circumferential face of the second rotating drum is formed into a substantially smooth surface, without a recessed portion that has a depth deeper than a thickness of the absorbent body when receiving the absorbent body from the first rotating drum, and
the belt member moves along the outer circumferential face together with the moving holding surface, at least while making the thickness thin, and the belt member is sucked towards the outer circumferential face using the suction air from the suction hole, and sandwiches and presses the absorbent body with the holding surface on the outer circumferential face.

9. A method that makes a thickness of an absorbent body thin according to any of claims 1 to 7, wherein
the holding surface is formed on one side of a moving member which moves along the travel path, and on an opposite surface of the holding surface of the moving member is oppositely positioned a space which enables the sucking of air through the suction hole on the holding surface,
the space is defined into a plurality of zones along the travel path, and an air pressure of a zone corresponding to the predetermined range of the plurality of zones is lower than that of a zone corresponding to a position on a downstream side of the predetermined range, and
an air pressure of a zone corresponding to a downstream end in the predetermined range is set to a value between
an air pressure of a zone other than a zone corresponding to the downstream end of the zones corresponding to the predetermined range and
an air pressure of a zone corresponding to a position on a downstream side of the predetermined range.

10. An apparatus which makes a thickness of an absorbent body, including a liquid absorbent fiber and a superabsorbent polymer, thin, the apparatus comprising:
a first apparatus which transports the absorbent body by having a holding surface which moves while holding the absorbent body on the holding surface with suction air from a suction hole on the holding surface; and
a second apparatus which makes the thickness of the absorbent body thin by having a belt member which is sucked towards the holding surface using the suction air from the suction hole, in a predetermined range of a travel path of the holding surface, the belt member being arranged opposing the holding surface along the travel path and being arranged movably along the travel path over the predetermined range of the travel path, and being arranged to sandwich the absorbent body between the holding surface and the belt member.
